(19) Europäisches Patentamt / European Patent Office / Office européen des brevets

(11) **EP 3 219 334 B1**

(12) **EUROPEAN PATENT SPECIFICATION**

(45) Date of publication and mention
of the grant of the patent:
**08.05.2019 Bulletin 2019/19**

(21) Application number: **15858670.1**

(22) Date of filing: **11.11.2015**

(51) Int Cl.:
*A61L 15/00* (2006.01)     *A61F 13/00* (2006.01)
*A61F 13/512* (2006.01)

(86) International application number:
**PCT/JP2015/081695**

(87) International publication number:
**WO 2016/076346 (19.05.2016 Gazette 2016/20)**

(54) **PERFORATED PLASTIC FILM**

PERFORIERTE KUNSTSTOFFFOLIE

FILM EN PLASTIQUE PERFORÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.11.2014   JP 2014229488
12.11.2014   JP 2014229490**

(43) Date of publication of application:
**20.09.2017   Bulletin 2017/38**

(73) Proprietor: **Asahi Kasei Kabushiki Kaisha
Tokyo 100-0006 (JP)**

(72) Inventors:
• **KUBOTA, Mio
Tokyo 101-8101 (JP)**
• **IRIYA, Masaru
Tokyo 101-8101 (JP)**
• **TOMONO, Masaki
Tokyo 101-8101 (JP)**

(74) Representative: **D Young & Co LLP
120 Holborn
London EC1N 2DY (GB)**

(56) References cited:
WO-A1-2012/096020     JP-A- H 042 499
JP-A- H0 586 216     JP-A- S62 148 246
JP-A- S62 148 246     JP-A- 2002 519 117
JP-A- 2005 178 365     JP-A- 2007 089 493
JP-A- 2014 237 810     US-A- 4 499 896

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 219 334 B1

## Description

Technical Field

The present invention relates to a perforated plastic film having a large number of through holes. The present invention particularly relates to a plastic film that is less irritating to the skin (low skin irritation) and is suitably used for skin protection.

Background Art

Films of plastics such as polyurethane have conventionally been used for skin protection such as for prevention of wounds and for treatment of wounded sites. Attaching a thin plastic film to the skin is known to allow for preventing body regions, such as the sacral region and heel region which are continuously exposed to a pressure from the body, from being reddened and then wounded due to friction or rubbing against linen or for protecting a lightly-wounded site.

However, plastic films have the disadvantages of being poor at appropriately releasing moisture evaporated from human skin and of causing the skin to become sweaty, macerated, and weakened and suffer a wound in the macerated site. To overcome these disadvantages, porous films with improved moisture permeability have been proposed.

For example, Patent Literature 1 proposes a wound protection material including: a fiber assembly with a metal vapor-deposited thereon; a fiber assembly for absorbing body fluids; and a microporous film having water repellency. Patent Literature 1 states that the diameter of the micropores typically ranges from 100 Å to 100 $\mu$m and the porosity typically ranges from 5 to 90%.

Patent Literature 2 proposes a sheet material for a wound covering material, the sheet material including a water vapor-permeable polymer material and a porous polymer material. Patent Literature 2 states that a water vapor permeability of 130 g/m$^2$·hr or more is achieved by a combination of a porous polymer material having a porosity of 40% or more with a silicone layer having a thickness of 20 $\mu$m or less.

Patent Literature 3 proposes using a soft elastomer film having holes in combination with a pad made of a gas-permeable fabric to form a wound bandage, and states that the holes have unsharp edges, which cause less damage to the tissue of a wound.

Citation List

Patent Literature

Patent Literature 1: Japanese Patent Laid-Open No. 62-275456
Patent Literature 2: Japanese Patent Laid-Open No. 5-261145
Patent Literature 3: Japanese Patent Laid-Open No. 63-183055

US 4 499 896 A discloses a wound dressing having a significant amount of exudate comprising a conformable, moisture vapor-permeable, liquid water-impermeable first layer containing at least one hole therein through which exudate can pass, and an imperforate, conformable, liquid water-permeable, moisture vapor-permeable second layer attached to the first layer and overlaying the hole or holes in the first layer and forming a reservoir into which the wound exudate can pass and from which the exudate can evaporate.

JP S62 148246 A discloses a resin film whose air permeability at 20°C is 30-10,000 sec and moisture vapor permeability at 20°C is 50-10,000 g/m$^2$.24hr.atm, obtained by a method wherein holes whose diameters are 50-600 $\mu$m are perforated through a heat-shrinkable resin film at the rate of 10-250 pieces/cm$^2$ and the heat-shrinkable resin film is shrunk 10-70 % by area by applying heat treatment to the heat-shrinkable resin film.

JP 2014 237810 A discloses a perforated film provided with a plurality of holes, wherein each of the holes includes a first notch provided on a plurality of first imaginary lines extending along a first direction, and a second notch extending along a second direction and a second incision made on a plurality of second imaginary lines, wherein the first direction is different from a direction perpendicular to the width direction and the width direction.

JP 2007 089493 A discloses a thermoplastic resin film that has a plurality of fine pores obtained by punching with a needle having a spinous process and has a moisture permeability of 500 g/m$^2$.24 hr or more.

JP H05 86216 A discloses production of a porous plastic film in which a plastic film is brought into contact with one electrode made of an embossing roll having irregularities on the surface, a discharge treatment is applied to the plastic film between the one electrode and the other electrode forming an opening in a portion contacting the porous plastic film.

Summary of Invention

Technical Problem

In the conventional techniques described in Patent Literatures 1 and 2 which employ a porous film, however, the moisture permeability of the film is difficult to control. That is, it is difficult to reduce the skin irritation by enabling the film to exhibit an appropriate level of moisture permeability which achieves the release of the moisture from the skin while also preventing the skin from being macerated and from being excessively dried.

The conventional technique described in Patent Literature 3 employs a soft elastomer as a film material to prevent the edges of holes from becoming sharp. This in turn increases the coefficient of friction on the skin so that the skin irritation cannot be low.

It is therefore an object of the present invention to provide a plastic film having an appropriate level of moisture permeability and having a low skin irritation potential.

Solution to Problem

Through a detailed study aimed at solving the above problems, the present inventors have discovered that a desired level of moisture permeability can easily be achieved by using a film made of any polymer material when the film is a plastic film having a large number of through holes formed by perforation, which is different from a so-called porous film having a large number of discontinuous micropores therein.

The present inventors have completed the present invention by finding that when such a plastic film having a large number of through holes has a moisture permeability of from 300 to 3500 $g/m^2 \cdot 24$ hr, the film has a low skin irritation and can thus be used for skin protection.

Specifically, the present invention is as follows.

[1] A perforated plastic film consisting of a plastic film having a large number of through holes, the perforated plastic film having a moisture permeability of 300 to 3500 $g/m^2 \cdot 24$ h.

[2] The perforated plastic film according to [1], wherein an average hole diameter of the large number of through holes is 5 to 300 $\mu$m, and a opening area ratio in the plastic film is 0.0003 to 4.5%.

[3] The perforated plastic film according to [1] or [2], wherein a effective opening area ratio in the large number of through holes is 80 to 100%.

[4] The perforated plastic film according to any of [1] to [3], wherein the plastic film has a friction coefficient of 1.7 or less.

[5] The perforated plastic film according to any of [1] to [4], wherein the plastic film comprises a polyethylene resin.

[6] The perforated plastic film according to [5], wherein the plastic film comprises polyethylene having a gel fraction of 10 to 60 mass%.

[7] The perforated plastic film according to any of [1] to [6], wherein the perforated plastic film is torn in a machine direction under tearing in the machine direction, is torn in a transverse direction under tearing in the transverse direction, and is torn in either the machine direction or the transverse direction under tearing in a direction at 45° to the machine direction.

[8] The perforated plastic film according to [7], wherein an acute angle formed between a tearing direction and a cut line under tearing in the direction at 45° to the machine direction is 30 to 60°, and a tear strength exhibited under tearing in the direction at 45° to the machine direction is 10 g or less.

[9] The perforated plastic film according to any of [1] to [8], being for the purpose of skin protection.

[10] A method for producing a perforated plastic film, the method comprising: a step of preparing a plastic film: that is torn in a machine direction under tearing in the machine direction, is torn in a transverse direction under tearing in the transverse direction, and is torn in either the machine direction or the transverse direction under tearing in a direction at 45° to the machine direction; that has an acute angle formed between a tearing direction and a cut line under tearing in the direction at 45° to the machine direction is 30 to 60°; and that has a tear strength exhibited under tearing in the direction at 45° to the machine direction is 10 g or less; and a perforation step of forming a large number of through holes in the plastic film.

[11] The method for producing a perforated plastic film according to [10], wherein the step of preparing the plastic film comprises a step of cross-linking the plastic film with electron beam.

[12] A composite product comprising a non-woven fabric and the perforated plastic film according to any of [1] to [8].

[13] A wound product comprising a core cylinder and the perforated plastic film according to any of [1] to [8], the perforated plastic film being wound on the core cylinder.

Advantageous Effects of Invention

The present invention can provide a plastic film having an appropriate level of moisture permeability and having a low skin irritation.

Brief Description of Drawings

Figure 1 is a diagram illustrating a diagonal tear test.
Figure 2 is a schematic diagram of a main part of a roll cutter device.
Figure 3 is an overall schematic diagram of a roll cutter device.
Figure 4 is a plan view of the perforated plastic film.

Description of Embodiments

An embodiment of the present invention (which will hereinafter be referred to as "present embodiment") will now be described in detail.

(Perforated plastic film)

The perforated plastic film according to the present invention is a plastic film having a large number of through holes and having a moisture permeability of from 300 to 3500 $g/m^2 \cdot 24$ h.

The moisture permeability as defined herein refers to a moisture permeability determined by converting the value of the mass of water vapor passing through the film at a temperature of 40°C and a humidity of 50% measured by a moisture permeability test method described later into a value per 1 $m^2$ (film area)·24 hours.

"[A] large number of through holes" refers to from 10 to 40000 holes/100 $cm^2$.

Various parameters are available for evaluating the water vapor permeability. For example, Patent Literature 2 described above employs a measurement method in which a sample is immersed directly in water. However, a parameter measured by such a method involving direct immersion of a sample in water is not suitable for the purpose of evaluating the water vapor permeability of the sample in terms of whether the sample has a low potential to cause skin maceration or dryness when attached to the skin, since the measurement environment used in the method greatly differs from actual use conditions. Thus, when such a parameter is employed for setting the water vapor permeability in a predetermined range (such as a range corresponding to the range of values of the so-called rate of water vapor transpiration from wounded surfaces), the actual skin irritation is considered not to be sufficiently low.

By contrast, the moisture permeability as employed in the present invention is determined by measuring the mass of water vapor passing through a sample in a normal ambient environment (at a temperature of 40°C and a humidity of 50%), and this measurement environment is similar to actual conditions of the use of a skin protection film. The moisture permeability as employed in the present invention is thus suitable for evaluating the properties (lowness of the potential to cause maceration or dryness) of a film for use in skin protection. A detailed study by the present inventors has revealed that when the moisture permeability of a plastic film is set in the range of from 300 to 3500 $g/m^2 \cdot 24$ hr, moisture evaporated from human skin can be appropriately released to prevent the skin from becoming sweaty and macerated or excessively dry and thus to reduce the skin irritation, and that this can be achieved without adversely affecting the below-described cutting properties or causing a physical irritation due to a film friction which is generated when the film slides over the skin or wounded area.

The moisture permeability is more preferably from 300 to 2000 $g/m^2 \cdot 24$ h. With the moisture permeability set within this range, the skin can be maintained under favorable moisture conditions so that the skin irritation can be further reduced. The moisture permeability is still more preferably from 300 to 1800 $g/m^2 \cdot 24$ h and most preferably from 600 to 1700 $g/m^2 \cdot 24$ h.

The moisture permeability of the perforated plastic film according to the present embodiment is the total of the moisture permeability attributed to water passing through the through holes and the moisture permeability attributed to water diffusing in and passing through the plastic portion. The former is considered to depend on the hole openings area rate, while the latter is considered to depend on the material properties (such as water vapor permeability) and thickness of the plastic film.

The moisture permeability may be adjusted based on the material properties and/or thickness of the film and/or the opening area ratio.

It is preferable to adjust the moisture permeability based on the opening area ratio, because this makes it possible to select a film material in consideration of properties other than the water vapor permeability and increase the variety of choices of film materials.

The openings of the large number of through holes of the perforated plastic film may be identical or different. The average hole diameter is preferably from 5 to 1000 $\mu$m and more preferably from 5 to 300 $\mu$m.

In the present invention, the "hole diameter" refers to the diameter of the opening in the film plane. However, the opening is not necessarily in the shape of an exact circle. Thus, in the present invention, the "hole diameter" is defined to refer to the diameter (circle equivalent diameter) of an exact circle having an area equal to the area of the opening as viewed in an image obtained by observing the opening directly from above with an optical microscope (with the target observation field set parallel to the film plane). When there is a perforation defect in an opening, the opening of such a defective portion is also regarded as one of the openings.

The device and software used in image processing for measuring the hole diameter are not particularly limited, and can be a known device and software. In the present embodiment, the average hole diameter of the film can be measured as follows.

An optical microscope (such as VHX-900 manufactured by KEYENCE CORPORATION) is set to an observation magnification of 100 times and an observation field of 1 cm square.

First, five target observation fields are randomly selected in the first surface of the film. For each target observation field, the hole diameters of all of the openings existing in the target observation field are measured and the average of the measured diameters is calculated. This operation is repeated five times, and then the average of the five average values obtained is calculated as the average hole diameter of the first surface.

Next, the film is turned upside down, and five target observation fields are randomly selected in the second surface of the film. The average of average values obtained for the five target observation fields is calculated as the average hole diameter of the second surface.

The smaller of the average hole diameters of the first and second surfaces is employed as the average hole diameter of the film.

When the average hole diameter is in the above range, entry of water or foreign matters can be prevented to maintain cleanliness. In addition, sweat, sebum, scurf etc. are less likely to clog the holes, and water vapor generated by evaporation from skin is readily released to the outside of the film, so that the skin can avoid becoming sweaty and macerated. Furthermore, the skin can maintain an appropriate level of moisture without becoming excessively dry.

The average hole diameter is more preferably from 5 to 250 $\mu$m, even more preferably from 7 to 200 $\mu$m, and most preferably from 10 to 150 $\mu$m.

The opening area ratio in the perforated plastic film ((the total of the areas of the openings/the area of the film) $\times$ 100) can be determined as appropriate depending on the material forming the film and on the desired moisture permeability, and is generally preferably from 0.0003 to 4.5%. When the opening area ratio is in this range, the desired moisture permeability can be achieved without a decrease in the strength of the film. When the film material used is any of the materials mentioned as specific examples later or has a water vapor permeability same as those of the materials mentioned as specific examples, an appropriate level of moisture permeability as described above can be obtained by adjusting the opening area ratio. The opening area ratio is more preferably 0.0015 to 2.0%, even more preferably 0.003 to 2.0%, and most preferably 0.01 to 1.5%.

In the present embodiment, the opening area ratio in the film can be measured as follows, similarly to the average hole diameter.

Five target observation fields are randomly selected in the first or second surface of the film whichever has a smaller average hole diameter. For each target observation field, the areas of all of the openings existing in the target observation field are measured, the average of the measured areas is calculated and, in addition, the number of the openings in the target observation field is counted. The average of the areas of the openings and the number of the openings are multiplied to obtain a total opening area. This operation is repeated five times, then the sum of the five values of the total opening area is divided by 5 cm$^2$ corresponding to the sum of the areas of the target observation fields, and the resulting value is employed as the opening area ratio of the film.

The opening area ratio can be controlled to a desired value by adjusting the average diameter or perforation density of the through holes formed by perforation in the film.

In the present embodiment, the effective opening area ratio of the through holes in the perforated plastic film is preferably from 80 to 100%. When the effective opening area ratio is in this range, physical irritation due to film friction generated when resin debris of a defectively perforated portion slides over the skin or wounded area can be decreased to reduce the irritation to the skin. The method for controlling the effective opening area ratio within the above range is not limited. The below-described method employing laser irradiation or a roll cutter is preferred, since, by using such a method, the effective opening area ratio within the above range can be achieved relatively easily.

In the present embodiment, the effective opening area ratio can be measured as follows.

Five target observation fields are randomly selected in the first or second surface of the film whichever has a smaller average hole diameter. For each target observation field, the areas of only the openings of perfectly perforated portions (such an area will hereinafter be referred to as "effective opening area") among all of the openings in the target observation field are measured. In this case, the areas of defectively perforated portions are not included in the effective opening areas. The total of the effective opening areas is divided by the total of the areas of all of the openings for each target observation field, and the average of the resulting values is calculated as the effective opening area ratio.

It is preferable for the perforated plastic film having a moisture permeability as specified in the present embodiment to have a friction coefficient of 1.7 or less, since such a film has a reduced skin irritation potential.

The friction coefficient as defined herein refers to the value of kinetic friction coefficient measured using the below-described test method in which the film is caused to slide on artificial skin at a speed of 1000 m/min over a distance of 80 mm. When the friction coefficient is in the range specified above, the physical irritation due to friction caused by the film sliding over the skin or wounded area can be decreased to reduce the skin irritation to such an extent that direct contact or attachment of the film to the skin causes no problem.

The friction coefficient is more preferably 1.3 or less, even more preferably 1.2 or less, and still even more preferably 1.0 or less.

The skin irritation reducing effect provided by controlling the friction coefficient to 1.7 or less is particularly significant when the moisture permeability is from 700 to 1500 $g/m^2 \cdot 24$ h, particularly from 800 to 1400 $g/m^2 \cdot 24$ h, more particularly from 900 to 1300 $g/m^2 \cdot 24$ h, and even more particularly from 1000 to 1200 $g/m^2 \cdot 24$ h.

The method for producing a perforated plastic film having a large number of through holes and having a friction coefficient within the above range is not limited. However, it is difficult to obtain a perforated plastic film having a low friction coefficient, since forming through holes by perforation in a plastic film is generally accompanied by formation of irregularities such as burrs and fins at the edges (peripheries) of the holes and such irregularities increase the friction coefficient. In fact, a perforated plastic film having a low friction coefficient as specified above has not been obtained thus far.

A detailed study conducted by the present inventors in this respect has provided the following findings a to c.

a. A plastic film highly oriented in two orthogonal directions is easy to perforate without formation of burrs etc.

Specifically, a plastic film is easy to perforate without formation of burrs etc. when the film is such that it is torn in the machine direction under tearing in the machine direction, is torn in the transverse direction under tearing in the transverse direction, and is torn in either the machine direction or the transverse direction under tearing in a direction at 45° to the machine direction.

In particular, the formation of burrs etc. is further reduced when the plastic film has an acute angle formed between a tearing direction and a cut line under tearing in the direction at 45° to the machine direction of from 30 to 60° and has a tear strength exhibited under tearing in the direction at 45° to the machine direction of 10 g or less.

b. When the plastic film comprises a polyethylene based resin and has a gel fraction (ASTM-D2765) of from 10 to 60 mass%, the plastic film is easy to perforate without formation of burrs etc.

c. When, for example, the below-described method using a pair of roll cutters or laser is employed as the perforation method, the formation of burrs etc. is reduced.

Thus, a perforated plastic film having a large number of through holes and having a friction coefficient of 1.7 or less can be produced by employing any or a combination of the conditions a to c specified above (i.e., by employing any one of the conditions a to c specified above, by employing a combination of two of the conditions a to c, or by employing all of the conditions a to c). It is particularly effective to employ the condition c, preferably in combination with either of the conditions a or b, more preferably in combination with both of the conditions a and b.

The moisture permeability of the perforated plastic film of the present embodiment can be freely set based on the opening area ratio. This is why the material of the perforated plastic film can be freely selected from a wide variety of materials without limitation on water vapor permeability etc. Specific examples of the material include: homopolymers of olefins such as polyethylene, polypropylene, polybutene, and poly-4-methylpentene; copolymers of two or more olefins; and copolymers of one or more olefins and another component different from olefins. The film preferably comprises a polyethylene based resin and particularly preferably consists of a polyethylene based resin. The polyethylene based resin as defined herein refers to a polymer compound containing an ethylene unit.

Such a polyethylene based resin is not particularly limited, and examples include: polyethylene; an ethylene-vinyl acetate copolymer; ethylene-aliphatic unsaturated carboxylic acid copolymers such as an ethylene-acrylic acid copolymer and an ethylene-methacrylic acid copolymer; and ethylene-aliphatic unsaturated carboxylic acid ester copolymers such as an ethylene-methyl acrylate copolymer, an ethylene-methyl methacrylate copolymer, an ethylene-ethyl acrylate copolymer, an ethylene-ethyl methacrylate copolymer, an ethylene-butyl acrylate copolymer, and an ethylene-butyl methacrylate copolymer.

These may be used alone or two or more thereof may be used in combination.

Preferred examples of the polyethylene based resins include ultralow-density polyethylene, high-pressure low-density polyethylene, linear low-density polyethylene, medium-density polyethylene, low-pressure high-density polyethylene, and ethylene-vinyl acetate copolymer.

The polyethylene based resin may include a known plasticizer where necessary. The type of the plasticizer is not limited. The plasticizer preferably has a low skin irritation, and specific examples include citric acid esters such as acetyl tributyl citrate, dimethyl phthalate, diethyl phthalate, dioctyl phthalate, glycerin, glycerin esters, waxes, liquid paraffins, phosphoric acid esters, and epoxidized soybean oils. Preferred are glycerin fatty acid esters, sorbitan fatty acid esters, polyoxyethylene fatty acid alcohol ethers, polyoxyethylene glycerin fatty acid esters, and polyoxyethylene sorbitan fatty acid esters.

These may be used alone or two or more thereof may be used in combination.

A plastic film comprising such a polyethylene based resin has a low potential to cause chemical irritation to the skin and, in addition, is so flexible that the film conforms to the skin and thus causes less physical damage to the skin. This can result in a reduction in skin irritation. Such a film is preferred also in that perforation of the film causes less formation of burrs etc.

In the present embodiment, when the perforated plastic film comprises a polyethylene based resin, the gel fraction (ASTM-D2765) of the film is preferably from 10 to 60 mass%. As previously described, the study by the present inventors has revealed that a polyethylene based resin-containing film having a gel fraction in the above range can be perforated with reduced formation of burrs etc. at the edges of the holes. The gel fraction is more preferably from 15 to 50 mass% and even more preferably from 25 to 40 mass%.

The gel fraction is a commonly used as an index indicating the degree of crosslinking. To control the gel fraction to from 10 to 60 mass%, the polymer compound forming the film may be crosslinked. The method for the crosslinking is not limited. For example, a crosslinking agent may be used or the film may be subjected to radiation crosslinking (electron beam crosslinking) to adjust the gel fraction. The radiation crosslinking is preferred, since the radiation crosslinking, unlike chemical crosslinking using a crosslinking agent, allows direct crosslinking of the polymer compound forming the film without mediation of any crosslinking agent and thus allows crosslinking without addition of any additive, thereby providing a reduction in skin irritation.

In the present embodiment, it is preferable for the perforated plastic film to be highly stretched in two orthogonal directions.

Specifically, it is preferable that the perforated plastic film be torn in the machine direction (MD direction in production of the film) under machine direction tearing, torn in the transverse direction (direction orthogonal to the machine direction, TD direction) under transverse direction tearing, and torn in either the machine direction or transverse direction under tearing in a direction at 45° to the machine direction.

A plastic film having such a feature can be perforated without much formation of burrs etc. Consequently, the resulting perforated plastic film can have a low friction coefficient. It also becomes possible to cut the perforated plastic film into any size by hands in such a manner that the cut surface has a straight profile.

The expressions "torn in the machine direction" and "torn in the transverse direction" as used herein mean that an acute angle formed between the machine direction or transverse direction and a cut line (the direction of cut) is from 0 to 15°. The term "cut line" as used herein refers to a line connecting the tearing start point and the tearing end point (intersections between the edges of a test piece of the film and the line made by actual tear). The acute angle formed between the machine direction or transverse direction and the cut line is more preferably from 0 to 10° and even more preferably from 0 to 5°.

For the evaluation of tearing, a tear test is conducted in the same manner as tear test method B specified in JIS K 7128 (Elmendorf method), and the acute angle formed between the machine direction or transverse direction and the direction of cut is measured.

The size of the test piece is 60 × 60 mm, and the slit length is 10 mm. When the evaluation is made for tearing in a direction at 45° to the machine direction of the film, the test piece is obtained from an original film along the direction at 45° to the machine direction.

Though the result of the evaluation of tearing of the film remains substantially the same before and after perforation, the result obtained for the perforated plastic film (after perforation) is used for the evaluation in the present embodiment.

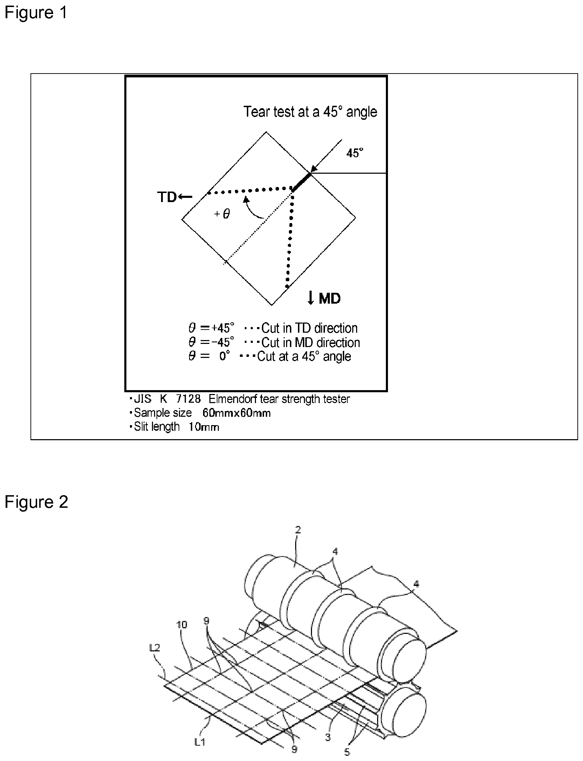

In the present embodiment, the acute angle formed between the tearing direction and the cut line when the film is subjected to tearing in the direction at 45° to the machine direction is preferably from 30 to 60°, more preferably from 35 to 55°, and even more preferably from 40 to 50°. For example, if the acute angle formed between the tearing direction and the cut line when the film is subjected to tearing in the direction at 45° to the machine direction of the film is 45°, then the direction of actual tear coincides with the machine direction or transverse direction (see Figure 1).

When the angle between the tearing direction and the cut line is in the above range, the film is likely to be successfully perforated with reduced formation of burrs etc. and is also likely to become easier to cut straight by hands.

The angle between the tearing direction and the cut line can be controlled based on the conditions of stretching in film production and on the degree of orientation created by stretching. When the film is biaxially stretched at a higher stretching ratio and at a lower stretching temperature, the degree of orientation of molecules becomes higher, and thus the molecular chain becomes more oriented in the MD and TD directions. When such a film is subjected to tearing, the film is likely to be torn in the direction in which the molecules are highly oriented. That is, the film is more likely to be torn in the MD and TD directions and is less likely to be torn in another direction such as the direction at 45° to the MD direction. Thus, when subjected to tearing in the direction at 45° to the machine direction, the film can be torn in the MD or TD direction. In cutting such a film wound in a roll on a core cylinder or the like with hand by drawing out a portion of the film from the core cylinder, once an initial cutout is formed, for example, by pressing a finger on the film while applying tension in the drawing direction, the film can be easily cut in the transverse direction of the roll by extending the cutting in the transverse direction starting from the initial cutout.

In the present embodiment, the tear strength exhibited under tearing in the direction at 45° to the MD direction of the film is preferably 10 g or less, more preferably 1 to 8 g, and even more preferably 2 to 6 g.

When the tear strength in the direction at 45° to the MD direction is in the above range, the cutting properties of the film in the MD direction and TD direction are likely to be further improved, and the film is likely to be successfully perforated with reduced formation of burrs etc. When the tear strength is 10 g or less, the film can easily be cut by hands.

The tear strength exhibited under tearing in the direction at 45° to the MD direction of the film, as defined herein, refers to a tear strength measured when torn in a tear test conducted in the same manner as tear test method B specified in JIS K 7128 (Elmendorf method).

The tear strength exhibited under tearing in the MD direction of the film is preferably 10 g or less, more preferably 1 to 8 g, and even more preferably 2 to 6 g. When the tear strength in the MD direction is in the above range, the hand-cutting properties of the film in the MD direction are likely to be further improved, and the film is likely to be successfully perforated with reduced formation of burrs etc. If the tear strength is more than 10 g, the film cannot easily be cut by hands.

The tear strength exhibited under tearing in the TD direction of the film is preferably 10 g or less, more preferably 1 to 8 g, and even more preferably 2 to 6 g. When the tear strength in the TD direction is in the above range, the hand-cutting properties of the film in the TD direction are likely to be further improved, and the film is likely to be successfully perforated with reduced formation of burrs etc. If the tear strength is more than 10 g, the film cannot easily be cut by hands.

In the present embodiment, the thickness of the perforated plastic film is preferably from 5.0 to 40.0 $\mu$m, more preferably from 5.0 to 20.0 $\mu$m, and even more preferably from 5.0 to 15.0 $\mu$m. When the thickness is 5.0 $\mu$m or more, the film is likely to be resistant to breakage. When the thickness is 40 $\mu$m or less, the cutting properties are likely to be further improved.

(Laminated product comprising perforated plastic film)

The perforated plastic film of the present embodiment may be used to form a laminated product by laminating the film with another porous film, a non-woven fabric, or paper or by applying a functional layer such as an adhesive layer or water-repellent layer on the perforated plastic film. When the laminated product is used for skin protection, the perforated plastic film is preferably placed on the side that is to be in contact with skin.

In this case, the moisture permeability of the entire laminated product also preferably falls within the range of from 300 to 3500 g/m$^2$·24 h.

(Method for using the perforated plastic film)

When the perforated plastic film is used for skin protection, the film can be cut into an appropriate size as necessary, overlaid on skin to cover a wounded area (or an area for which prevention of wounds is desired), and secured on the skin with the aid of a surgical tape or the like attached onto the film. An ointment such as petrolatum may be applied to a wounded area of skin (or an area for which prevention of wounds is desired), and then the film may be overlaid on the applied ointment and attached to the area by means of the adhesive effect of the ointment.

Alternatively, the film cut into an appropriate size is laminated with a frame-shaped adhesive layer to form a laminated product, and the laminated product can be used by attaching it to the skin so that a wounded area of the skin (or an area for which prevention of wounds is desired) lies within the frame defined by the frame-shaped adhesive layer.

(Method for producing perforated plastic film)

The method for producing the perforated plastic film of the present embodiment is not limited. For example, the perforated plastic film can be produced by a production method of the present embodiment that comprises a step of preparing a base film and a perforation step. The production method of the present embodiment may optionally further comprise an electron beam crosslinking step and/or a stretching step as a part of the step of preparing a base film.

The step of preparing a base film is a step of preparing a base film to be processed into the perforated plastic film of the present embodiment and can be accomplished, for example, by employing a known film production method.

This step can comprise, for example, the steps of: extruding a resin and a plasticizer into the form of a single-layer or multi-layer original film through an annular die; cooling and solidifying the extruded original film; and stretching the original film cooled and solidified.

The stretching step is a known step of uniaxially or biaxially stretching the original film. The method for stretching is not limited. Biaxial stretching can be preferably used. Sequential or simultaneous biaxial stretching or inflation biaxial stretching can be more preferably used. Particularly preferred is inflation biaxial stretching. Biaxial stretching is likely to further improve the cutting properties of the film.

The stretching ratio is preferably from 5.0 to 12, more preferably from 5.5 to 11, and even more preferably from 6.0 to 10 for both of the MD and TD directions. When the stretching ratio is in the above range, the degree of orientation of the polymer compound forming the film is likely to be so high as to result in a film that has good hand-cutting properties and suffers less from defective tearing (i.e., a film that is torn in the MD direction under MD direction tearing, torn in the TD direction under TD direction tearing, and torn in either the MD direction or TD direction under tearing in the direction at 45° to the MD direction).

The stretching ratio in the TD direction refers to a ratio expressed as (film width after stretching)/(parison width before stretching), and the stretching ratio in the MD direction refers to a ratio expressed as (line speed after stretching)/(line speed before stretching).

The area stretching ratio is preferably 5 to 70 and more preferably 20 to 60. When the area stretching ratio is 5 or more, the cutting properties are likely to be further improved. When the area stretching ratio is 70 or less, the dimensional change of the product is likely to be reduced.

The stretching temperature is preferably equal to or lower than a temperature 60°C higher than the melting point of the material constituting the film, more preferably equal to or lower than a temperature 40°C higher than the melting point, and even more preferably equal to or lower than a temperature 30°C higher than the melting point. When the stretching temperature is in the above range, the degree of orientation of the polymer compound constituting the film is likely to be so high that the hand-cutting properties are further improved.

When the plastic film comprises a polyethylene based resin, it is preferable to perform crosslinking process by irradiating the parison or the stretched film with a radioactive ray. The radioactive ray used in the radiation crosslinking process is not particularly limited, and examples include ionizing radioactive rays such as ultraviolet ray, electron beam, X ray, $\alpha$ ray, $\beta$ ray, $\gamma$ ray, and neutron ray. Among these, electron beam is preferred. An example of the irradiation process using electron beam irradiation is to irradiate the entire parison or film with electron beam at an energy voltage of from 100 kV to 1 MV.

In terms of reducing the formation of burrs etc. in perforation, radiation crosslinking is preferably performed so that the film subjected to the crosslinking has a gel fraction, as specified in ASTM-D2765, of 10 to 60 mass%.

When the crosslinking is performed before stretching, the stretching can be done at a high stretching ratio. The stretching at a high stretching ratio leads to high degree of orientation of the chain of the polymer forming the film, thus enabling the film to be easily cut by hands without the help of any cutting tool.

The gel fraction of a film is commonly used as an index of the degree of crosslinking. Increasing the gel fraction allows stretching at an increased stretching ratio. However, too high a gel fraction is likely to make the stretching difficult. In the present embodiment, therefore, the gel fraction is more preferably from 15 to 50 mass% and even more preferably from 25 to 40 mass%.

The perforation step is a step of forming small holes in the base film. The method for perforation is not limited. For example, any of known methods such as hot needle puncture and laser irradiation can be used.

The average hole diameter can be adjusted by setting the diameter of the hot needle or the output power of the laser appropriately depending on the thickness or material of the film.

In the present embodiment, the hole diameter and hole density and hence the opening area ratio can be freely adjusted by controlling the conditions of perforation (such as the laser output power, needle diameter, and perforation density) in the perforation step. Thus, the moisture permeability of the perforated plastic film can easily be set.

When a laser is used for the perforation in the present embodiment, it is preferable to place a base such as paper or another film on the film surface opposite to the surface to be irradiated with the laser, since in this case the periphery of each hole formed by the irradiation is loosely attached to the base so that the process can be accomplished without closure of the perforated holes due to thermal contraction.

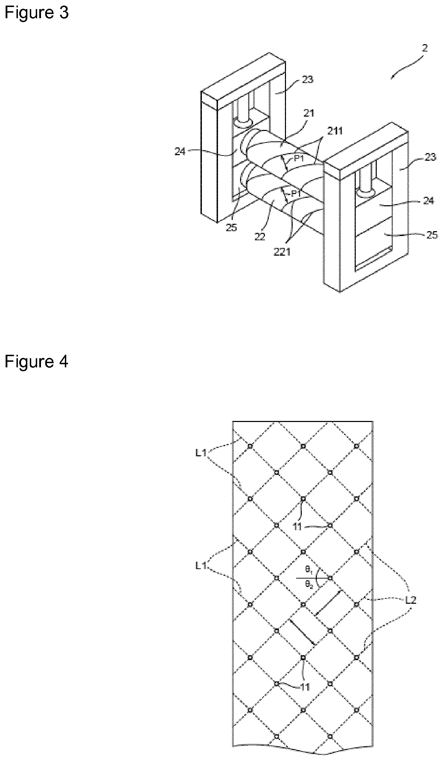

The perforation step may employ a perforation device as shown in Figure 2 or Figure 3 which includes a pair of roll cutters differing in the arrangement pattern of cutting blades.

For example, in the perforation device shown in Figure 2, a film 10 passing between rolls 2 and 3 is exposed to pressures from above and below simultaneously in the vicinity of points where cutting blades 4 arranged on the roll 2 and cutting blades 5 arranged on the roll 3 cross each other, i.e., points where the lines L1 and the lines L2 cross each other in plan view. This allows the film 10 to be perforated at the points 9 where the cutting blades 4 and the cutting blades 5 cross each other, thus resulting in the formation of through holes.

The perforation using such a roll cutter causes less formation of irregularities such as burrs and fins at the edges of the holes, and thus allows easy production of a perforated plastic film that maintains a flat surface and a low friction coefficient after the formation of the holes.

The perforated plastic film formed by perforation using such a roll cutter has a flat surface. Thus, when wound into a roll, the film is less likely to have wrinkles, humps and the like and can be in a roll with good appearance, as well as being capable of avoiding being marked with winding traces. A good-quality product is consequently obtained.

The method for producing a perforated plastic film according to the present embodiment may further include surface treatment such as corona treatment or plasma treatment, printing, or application of an adhesive.

In the present embodiment, the perforated plastic film can be formed into a roll wound on a core cylinder. Forming the film into a roll improves the handling of the film and allows the film to be easily cut into a piece having a desired area. In particular, a portion of the film drawn out from the roll can easily be cut out straight by hands when the film is such that it is torn in the machine direction under tearing in the drawing direction, torn in the transverse direction under tearing in the transverse direction, and torn in either the drawing direction or transverse direction under tearing in the direction at 45° to the drawing direction, such that the acute angle formed between the tearing direction and the cut line under tearing in the direction at 45° to the drawing direction is 30 to 60°, and such that the tear strength exhibited under tearing in the direction at 45° to the drawing direction is 10 g or less.

The material of the core cylinder is not limited. For example, a core cylinder made of paper, plastic, metal, or the like can be used. The core cylinder may be in the form of a circular tube having a hollow interior or a non-hollow circular column. When the core cylinder is hollow, it is preferable for the core cylinder used to have a thickness of 0.5 mm or more so as to withstand a force (grip force) applied when the user grips and pulls a portion of the film roll by one hand and then makes an initial cut with a finger of the other hand while applying a tension in the drawing direction. The upper limit of the thickness is not particularly defined.

The diameter of the core cylinder is not limited and can be, for example, from 10 mm to 50 mm.

[Examples]

Next, the present invention will be described in more detail by way of Examples, Reference Examples and Comparative Examples. The present invention is not limited to Examples described below. In Examples, Reference Examples and Comparative Examples, the moisture permeability, the average hole diameter, the opening area ratio, the effective opening area ratio, the friction coefficient, the gel fraction, the diagonal tear, and the thickness were measured by the following methods.

(Moisture permeability)

The moisture permeability was evaluated by the following procedures using a measurement device identical to that as used in A-2 method (Water method) specified in JIS 1099 "Testing methods for water vapor permeability of textiles".

An amount of 30 ml of 40°C purified water was put in a 60-mm-diameter and 25-mm-deep aluminum cup heated beforehand to 40°C. A 70-mm-diameter test piece was placed on and concentrically with the cup in such a manner that the surface of the test piece having a smaller average hole diameter described below faced to the water. A packing and a ring were attached and fixed with a wing nut to prepare a test object. This test object was placed in a thermo-hygrostat set at 40 $\pm$ 2°C and 50 $\pm$ 5%RH and allowed to stand still for 8 hours at a place where the air flow speed at a height of 10 mm from the test piece was not more than 0.8 m/s. After that, the change in weight of the test object was measured. The moisture permeability was calculated by the following formula.

$$\text{Moisture permeability (g/m}^2\cdot\text{24 h)} = [\text{Weight change (g)/Moisture permeation area (m}^2)] \times 3$$

(Average hole diameter)

An optical microscope (VHX-900 manufactured by KEYENCE CORPORATION) was set to an observation magnification of 100 times and an observation field of view of 1 cm square. A target observation field was set 5 cm inward from one edge (edge in the MD direction) of the first surface of the film. The diameters of all of the openings in this target observation field were calculated, and their average was determined.

Furthermore, four other target observation fields were set at intervals of 1 m in the MD direction (feed direction) of the film from the above target observation field and, for each target observation field, the measurement of diameters of the openings and the calculation of their average were performed in the same way as above. The five average values were finally averaged, and the resulting value was employed as the average hole diameter of the first surface of the film.

The film was turned upside down, and the average hole diameter of the second surface was measured in the same manner as above. The smaller of the average hole diameters of the first surface and second surface was employed as the average hole diameter of the film.

(Opening area ratio)

In the same manner as in the above measurement of the average hole diameter, an target observation field to be observed using an optical microscope with a field of view of 1 cm square was set in the first surface or second surface of the film whichever had a smaller average hole diameter. The areas of all of the openings in this target observation field were measured, the average of the measured areas was calculated and, in addition, the number of the openings in the target observation field was counted. The average of the areas and the number of the openings were multiplied to calculate a total opening area.

A total of five target observation fields were set at intervals of 1 m in the MD direction of the film, and the same operation as above was carried out to calculate a total opening area for each target observation field. The sum of the five values of the total opening area was divided by 5 cm$^2$, which is the sum of the areas of the target observation fields, to obtain the opening area ratio of the film.

(Effective opening area ratio)

In the same manner as in the above measurement of the average hole diameter, an target observation field to be observed using an optical microscope with a field of view of 1 cm square was set in the first surface or second surface of the film whichever had a smaller average hole diameter. The areas of all of the openings in the target observation field were measured and the sum thereof was calculated, while the areas of only the openings of perfectly perforated portions (effective opening areas) were measured and the sum thereof was calculated. The areas of defectively perforated portions were not included in the effective opening areas.

A total of five target observation fields were set at intervals of 1 m in the MD direction of the film. For each target observation field, the same operation as above was carried out, and the total of the effective opening areas was divided by the total of the areas of all of the openings. The five resulting values were averaged.

(Friction coefficient)

A friction tester (TR-2) manufactured by Toyo Seiki Seisaku-Sho, Ltd. was used. A perforated film (having a length of 100 mm and a width of 64 mm) as a measurement object was attached to a metal rider having a friction face made of foam material and having a length of 100 mm, a width of 64 mm, and a weight of 200 g, and was caused to slide on artificial skin, Bioskin Plate Normal (manufactured by Beaulax Co., Ltd. and having a length of 195 mm and a width of 130 mm), at a speed of 1000 m/min over a distance of 80 mm to measure a dynamic friction coefficient.

(Gel fraction)

The measurement was performed according to ASTM-D2765 as follows.

The film was immersed in boiling paraxylene for 12 hours, and the mass fraction of the undissolved portion of the film as expressed by the formula below was employed as the gel fraction. The sample used was prepared by restoring the stretched film into a parison form by thermally contracting the stretched film at 140°C.

$$\text{Gel fraction (mass\%)} = (\text{Sample mass after immersion/Sample mass before immersion}) \times 100$$

(Diagonal tear test and cutting properties)

Figure 1 schematically illustrates the diagonal tear test (test for tearing properties in the direction at 45° to the MD direction) in the Examples.

The tear strength in the direction at 45° to the MD direction of the base film was measured using an Elmendorf tear strength tester (manufactured by Toyo Seiki Seisaku-Sho, Ltd.) complying with JIS K 7128 in the same manner as specified in JIS K 7128, except that a 60 cm × 60 cm film having a cut with a length of 1 cmt was made was subjected to tearing in the direction at 45° to the MD direction.

In the diagonal tear test, an acute angle formed between the direction in which the film was actually torn and the tearing direction (direction at 45° to the MD direction) was measured.

The evaluation of the cutting properties in the diagonal tear test was made by evaluating the acute angle formed between the direction in which the film was torn and the tearing direction (direction at 45° to the MD direction) according to the following evaluation criteria.

<Evaluation criteria>

◎: The acute angle formed between the direction in which the film was torn and the tearing direction was 40° or more and 50° or less, and the tear strength was from 2 to 6 g.

○: The acute angle formed between the direction in which the film was torn and the tearing direction was 30° or more and less than 40° or was more than 50° and 60° or less, and the tear strength was 10 g or less.

×: The acute angle formed between the direction in which the film was actually torn and the tearing direction was more than 60° or less than 30°, or the tear strength was more than 10 g.

(Tear test in MD direction and TD direction)

Tear test in the MD direction and TD direction of the base film was conducted using an Elmendorf tear strength tester (manufactured by Toyo Seiki Seisaku-Sho, Ltd.) according to JIS K 7128. A 60 cm × 60 cm film having a cut with a length of a 1 cm was prepared and subjected to the tear test in the MD direction and TD direction, and the evaluation was made according to the following criteria.

<Evaluation criteria>

○: The acute angle formed between the direction in which the film was torn and the tearing direction was 15° or less.

×: The acute angle formed between the direction in which the film was torn and the tearing direction was more than 15°.

(Film thickness)

The film thickness was measured according to ASTM E-252. Specifically, the measurement was carried out using TECLOCK US-26 manufactured by TECLOCK CORPORATION.

(Skin irritation)

Sample pieces having a shape of 5-cm-square were cut out from the film, and attached to the skin of the upper arm of 20 subjects having healthy skin with a surgical tape. The pieces of the film were peeled off 24 hours later, and the subjects were then left for 30 minutes. After that, the condition of the skin that was in contact with the film was evaluated as follows.

<Evaluation criteria>

◎: 19 or more of the subjects did not show maceration or suffer unpleasant sensation such as itching, and none of the subjects showed redness.

○: From 16 to 18 of the subjects did not show maceration or suffer unpleasant sensation such as itching, and none of the subjects showed redness.

△: From 10 to 15 of the subjects did not show maceration or suffer unpleasant sensation such as itching, and none of the subjects showed redness.

X: 9 or less of the subjects did not show maceration or suffer unpleasant sensation such as itching, or 1 or more of the subjects showed redness.

[Examples 1 to 4]

Glyceryl monooleate was added in an amount of 0.5 mass% to a polyethylene based resin (composition) containing LL (ethylene-1-octene copolymer, density = 0.926 g/cm$^3$, MI = 2.0 g/10 min) and LD (high-pressure low-density polyethylene, density = 0.921 g/cm$^3$, MI = 0.4 g/10 min) at a LL : LD ratio of 70 : 30, and the mixture was extruded into the form of a single-layer original film(Examples 1 and 4), a three-layer original film (Example 2), or a five-layer original film (Example 3) through an annular die, and the extrudate was then cooled and solidified with cool water to fabricate a tubular original film with a folding width of 120 mm and a thickness of 500 $\mu$m.

The original firm was introduced to an electron beam irradiation device and was subjected to crosslinking treatment by irradiating it with an electron beam accelerated to 500 kV in such a manner that the absorbed dose became 80 kGy.

The original film was introduced into a stretching machine, in which the original film was reheated, passed between two pairs of differential nip rolls, injected with air to form bubbles, and stretched by a factor of 8 in the MD direction and a factor of 6 in the TD direction (Examples 1 and 3) or by a factor of 6 in the MD direction and a factor of 6 in the TD direction (Example 2) to obtain double-ply films. In Examples 1, 3, and 4, the double-ply film was further subjected to a slit step of opening the double-ply film into a single film as a base film.

The obtained base film was perforated by laser irradiation to obtain a perforated plastic film (in Examples 1, 3, and 4) or by perforating the base film using a hot needle adjusted to an appropriate temperature in the range of 135 to 170°C in consideration of the gelatinization temperature and melting temperature of the resin (in Example 2).

The average hole diameter was controlled by adjusting the output power of the laser in Examples 1 and 3 and by adjusting the diameter of the hot needle in Example 2.

[Example 5]

A base film obtained in the same manner as that in Examples 1 and 3 (it should be noted that the extrudate was a single-layer film and the film was stretched by a factor of 8 in the MD direction and a factor of 6 in the TD direction) was perforated using a roll cutter device 2 including a pair of roll cutters 21 and 22 having different cutting-blade arrangement patterns in order to obtain a perforated plastic film.

Figure 3 shows the roll cutter device 2 which was used.

In the roll cutter device 2, the roll cutter 21 and the roll cutter 22 are circular tubular or columnar cutters and are arranged opposite to each other. The axis of the roll cutter 21 and the axis of the roll cutter 22 are parallel to each other, and the roll cutter 21 and the roll cutter 22 are spaced at a distance that allows them to hold an object to be perforated therebetween. Supports 24 are provided at both ends in the axial direction of the roll cutter 21, and the roll cutter 21 is supported on frames 23 via the supports 24 to be rotatable about its axis. Supports 25 are provided at both ends in the axial direction of the roll cutter 22, and the roll cutter 22 is supported on the frames 23 via the supports 25 to be rotatable about its axis. The roll cutter 21 and the roll cutter 22 rotate in conjunction with each other. The rotational direction C1 of the roll cutter 21 is opposite to the rotational direction C2 of the roll cutter 22.

Cutting blades 211 are arranged on the circumferential surface of the roll cutter 21. The cutting blades 211 are arranged at a pitch P1, and each cutting blade 211 is inclined at an angle of $\theta_1$ to the axial direction of the roll cutter 21 and is continuous in the circumferential direction of the roll cutter 21. On the circumferential surface of the roll cutter 22 there are arranged cutting blades 221. The cutting blades 221 are arranged at a pitch P2, and each cutting blade 221 is inclined at an angle of $\theta_2$ to the axial direction of the roll cutter 22 and is continuous in the circumferential direction of the roll cutter 22. The cutting blades 211 and cutting blades 221 are inclined at the same angle to the axial direction. The cutting blades 211 and 221 are discretely arranged at the pitch P1 or P2 in Figure 2; however, helical cutting blades may alternatively be employed.

Between the roll cutter 21 and roll cutter 22 of this roll cutter device 2, one end of the base film is inserted in the same manner as that shown in Figure 2. The cutting blades 211 of the roll cutter 21 come into contact with the upper surface of the base film, while the cutting blades 221 of the roll cutter 22 come into contact with the lower surface of the base film. In this state, the roll cutter 21 and roll cutter 22 are rotated in conjunction with each other to process the base film while conveying the film in the longitudinal direction of the film.

Figure 4 shows a plan view of the base film having passed between the pair of roll cutters 21 and 22 of the roll cutter device 2. The roll cutter device 2 causes the cutting blades 211 to be pressed against the upper surface of the base film so that lines L1 are left by the cutting blades 211 on the upper surface. The cutting blades 221 are pressed against the lower surface of the base film so that lines L2 are left by the cutting blades 221 on the lower surface. When viewed from the upper surface of the base film, the multiple lines L1 are arranged at the pitch P1 and extend in the longitudinal direction of the base film while being inclined at the angle of $\theta_1$ to the width direction of the base film. When viewed from the lower surface of the base film, the multiple lines L2 are arranged at the pitch P2 and extend in the longitudinal direction of the base film while being inclined at the angle of $\theta_2$ in a direction opposite to the inclination of the lines L1 with respect to the width direction of the base film.

Thus, holes 11 are sequentially formed in the base film at the points where the cutting blades 211 and cutting blades 221 cross each other, and a perforated film is fabricated.

[Example 6]

To 100 parts by mass of vinylidene chloride-vinyl chloride copolymer PVDC (vinylidene chloride monomer unit content/vinyl chloride monomer unit content = 80 mass%/20 mass%, mass-average molecular weight = 120,000) were added 3 parts by mass of dibutyl sebacate as an fatty acid ester, 4 parts by mass of acetyl tributyl citrate, and 2 parts by mass of epoxidized soybean oil as an epoxy compound, and they were mixed by a Henschel mixer for 5 minutes. The resulting mixture was extruded into a tube by a melt extruder, and the extruded tube was supercooled in a cool water bath at about 10°C. After that, the tube was passed through 35°C water and stretched by inflation biaxial stretching at a stretching temperature of 30°C by a factor of 3.0 in the MD direction and a factor of 4.0 in the TD direction. The resulting tubular film was folded with a pinch roll to obtain a flat, long base film of 10 $\mu$m thickness.

The base film was perforated using the roll cutter device 2 in the same manner as in Example 5, and thus a perforated plastic film was obtained.

[Example 7]

LD (high-pressure low-density polyethylene, density = 0.921 g/cm$^3$, MI = 0.4 g/10 min) was extruded into a tube by a melt extruder, then processed with bubble formation, and the extrudate was rolled up to prepare a base film.

Next, the base film was perforated using a hot needle adjusted to an appropriate temperature in the range of from 135 to 170°C in the same manner as in Example 2, and thus a perforated plastic film was obtained.

[Example 8]

Polypropylene was extruded by a melt extruder, and the extrudate was cooled and solidified. The extrudate was then reheated and stretched by sequential biaxial stretching by a factor of 4 in the MD direction and a factor of 8 in the TD direction to obtain a base film.

Next, the base film was perforated using a hot needle adjusted to an appropriate temperature in the range of from 180 to 220°C in the same manner as in Example 2, and thus a perforated plastic film was obtained.

[Example 9]

A base film was fabricated in the same manner as in Example 1, bonded to an olefin based spunbond non-woven fabric at the time of laser irradiation, and perforated by laser irradiation in the same manner as in Example 1. The base film and the non-woven fabric were combined in the course of the perforation, and thus a perforated plastic film laminate was obtained.

[Example 10]

A perforated plastic film was obtained in the same manner as in Example 5, except that, in the base film production,the extrudate was prepared in a three-layer and stretched by a factor of 6 in the MD direction and a factor of 6 in the TD direction.

[Example 11]

A perforated plastic film was obtained in the same manner as in Example 5, except that the extrudate was prepared in a five-layerin the base film production.

[Example 12]

Polyethylene terephthalate was extruded by a melt extruder, and the extrudate was subjected to sequential biaxial stretching to obtain a base film. The base film was perforated using roll cutters in the same manner as in Example 5, and thus a perforated plastic film was obtained.

[Example 13]

Nylon 6 was extruded by a melt extruder, and the extrudate was biaxially stretched to obtain a base film. The base film was perforated using roll cutters in the same manner as in Example 5, and thus a perforated plastic film was obtained.

[Example 14]

A base film made of LL/LD was obtained in the same manner as in Example 1.

Next, the base film was perforated using a hot needle adjusted to an appropriate temperature in the range of 135 to 170°C in the same manner as in Example 2, and thus a perforated plastic film was obtained.

[Comparative Example 1]

A base film was obtained in the same manner as in Example 7.

Next, the base film was perforated by a porous process, as described in Japanese Patent No. 1995281, in which the film was pressed against protrusions. A perforated plastic film was thus obtained.

[Comparative Example 2]

A tubular original film was fabricated in the same manner as in Example 1. The original film was introduced to an electron beam irradiation device and was subjected to crosslinking by irradiating it with an electron beam in such a manner that the absorbed dose was adjusted to result in a gel fraction of 61 mass%.

The original film was introduced into a stretching machine, in which the original film was reheated, passed between two pairs of differential nip rolls, and processed with air injection. However, formation of bubbles failed, and collection of the film was impossible.

[Comparative Example 3]

Polyethylene terephthalate was extruded by a melt extruder, and the extrudate was subjected to sequential biaxial stretching to obtain a base film. A perforated plastic film was obtained by perforating the base film using a hot needle adjusted to an appropriate temperature in the range of 150 to 220°C in consideration of the gelatinization temperature and melting temperature of the resin.

[Comparative Example 4]

Low-density polyethylene (density = 0.921 g/cm$^3$, MI = 0.4 g/10 min) was extruded into a tube by a melt extruder, followed by bubble formation and the extrudate was rolled up to prepare a base film. Next, the base film was perforated by a porous process, as described in Japanese Patent No. 1995281, in which the film was pressed against protrusions. A perforated plastic film was thus obtained.

Table 1 shows the various properties of the perforated plastic films produced in Examples and Comparative Examples as well as the results of the diagonal tear test and skin irritation sensory test conducted on the films.

[Table 1]

| | Material | Thickness | Moisture per-meability | Hole di-ameter | Opening area ratio | Effective opening area ratio | Friction co-efficient | Gel frac-tion | MD/TD direction tear test | | Diagonal tear test | | Effect | |
|---|---|---|---|---|---|---|---|---|---|---|---|---|---|---|
| | | | | | | | | | MD direc-tion | TD direc-tion | Cut direc-tion | Tear strength | Skin irri-tation | Cutting properties |
| | | μm | g/m²·24 h | μm | % | % | | % | | | Degrees | g | | |
| Example 1 | LL/LD | 8 | 850 | 100 | 1.1 | 95 | 0.92 | 33 | ○ | ○ | 38 | 3.6 | ◎ | ◎ |
| Example 2 | LL/LD | 40 | 1730 | 700 | 1.6 | 70 | 1.58 | 33 | ○ | ○ | 41 | 10 | ○ | ○ |
| Example 3 | LL/LD | 5 | 310 | 130 | 0.0002 | 90 | 0.71 | 33 | ○ | ○ | 37 | 3.7 | ○ | ◎ |
| Example 4 | LL/LD | 11 | 3000 | 200 | 4.4 | 92 | 0.88 | 25 | ○ | ○ | 37 | 3.6 | ○ | ◎ |
| Example 5 | LL/LD | 8 | 1050 | 11 | 0.016 | 85 | 0.92 | 28 | ○ | ○ | 38 | 3.7 | ◎ | ◎ |
| Example 6* | PVDC | 10 | 610 | 10 | 0.015 | 83 | 0.85 | - | ○ | ○ | 20 | 4.1 | ○ | ○ |
| Example 7* | LD | 10 | 330 | 10 | 0.01 | 85 | 0.91 | 0 | Not torn | Not torn | Not torn | Not torn | ○ | × |
| Example 8* | PP | 20 | 650 | 200 | 0.06 | 90 | 0.89 | - | ○ | ○ | 2 | 10 | ○ | × |
| Example 9* | LL/LD/Non-woven fabric | 8 | 850 | 100 | 1.1 | 95 | 0.92 | 33 | Not torn | Not torn | Not torn | Not torn | ○ | × |
| Example 10 | LL/LD | 11 | 750 | 20 | 0.005 | 85 | 0.91 | 33 | ○ | ○ | 40 | 3.6 | ○ | ◎ |
| Example 11 | LL/LD | 20 | 1350 | 35 | 0.03 | 84 | 0.72 | 33 | ○ | ○ | 39 | 6.5 | ○ | ○ |
| Example 12* | PET | 12 | 970 | 10 | 0.016 | 83 | 1.25 | - | ○ | ○ | 20 | 10 | △ | × |
| Example 13* | Ny | 20 | 710 | 40 | 0.01 | 83 | 0.64 | - | ○ | ○ | 21 | 20 | △ | × |
| Example 14 | LL/LD | 8 | 3200 | 500 | 7 | 77 | 1.4 | 33 | ○ | ○ | 38 | 3.6 | △ | ◎ |
| Comparative Example 1 | LD | 10 | 30 | 10 | 0.0002 | 72 | 0.6 | - | Not torn | Not torn | Not torn | Not torn | △ | × |
| Comparative Example 2 | LL/LD | - | - | - | - | - | - | 61 | - | - | - | - | - | - |
| Comparative Example 3 | PET | 40 | 3700 | 900 | 20 | 65 | 1.96 | - | × | × | 15 | 20 | △ | × |

(continued)

| | Material | Thickness | Moisture permeability | Hole diameter | Opening area ratio | Effective opening area ratio | Friction coefficient | Gel fraction | MD/TD direction tear test | | Diagonal tear test | | Effect | |
|---|---|---|---|---|---|---|---|---|---|---|---|---|---|---|
| | | | | | | | | | MD direction | TD direction | Cut direction | Tear strength | Skin irritation | Cutting properties |
| Comparative Example 4 | LD | 10 | 70 | 3 | 0.001 | 71 | 0.55 | - | Not torn | Not torn | Not torn | Not torn | × | × |

\* = Reference Example

Industrial Applicability

The plastic film for skin protection according to the present invention can be suitably used for a wide variety of skin protection purposes such as wound prevention, wound protection, wound treatment, and the like.

Specifically, the plastic film can be used, for example, as an adhesive plaster, a wound covering material, a dressing, a bandage, a surgical tape, a film for occlusive dressing therapy, a wrap for burn injury, a surgical covering and protecting material, a bedsore prevention film, a film for transdermal absorption materials, a film for wristbands for patient identification, a taping material, a supporter, a film for fixing of indwelling needles, or a film for patch tests.

The present application is based on Japanese Patent Applications (Japanese Patent Application No. 2014-229488 and Japanese Patent Application No. 2014-229490) filed with Japan Patent Office on November 12, 2014.

**Claims**

1.  A perforated plastic film consisting of a plastic film having from 10 to 40000 through holes/100 cm$^2$, the perforated plastic film having a moisture permeability of from 300 to 3500 g/m$^2$·24 h,
    wherein the plastic film consists of polyethylene having a gel fraction of from 10 to 60 mass%, or
    wherein the plastic film is stretched in a way that it is torn in a machine direction under tearing in the machine direction, in a transverse direction under tearing in the transverse direction, and in either the machine direction or the transverse direction under tearing in a direction at 45° to the machine direction,
    wherein "torn in a machine direction" and "torn in a transverse direction" mean that an acute angle formed between the machine direction or the transverse direction and a cut line is from 0 to 15°.

2.  The perforated plastic film according to claim 1, wherein the perforated plastic film is stretched in a way that it is torn in a machine direction under tearing in the machine direction, in a transverse direction under tearing in the transverse direction, and in either the machine direction or the transverse direction under tearing in a direction at 45° to the machine direction, and
    wherein the plastic film is stretched in a way that it has an acute angle formed between a tearing direction and a cut line under tearing in the direction at 45° to the machine direction of from 30 to 60°, and a tear strength exhibited under tearing in the direction at 45° to the machine direction of 10 g or less.

3.  The perforated plastic film according to claim 1 or claim 2, wherein an average hole diameter of the through holes is from 5 to 300 $\mu$m, and an opening area ratio in the plastic film is from 0.0003 to 4.5%.

4.  The perforated plastic film according to any one of claims 1 to 3, wherein an effective opening area ratio of the through holes is 80 to 100%.

5.  The perforated plastic film according to any one of claims 1 to 4, wherein the plastic film has a friction coefficient of 1.7 or less.

6.  The perforated plastic film according to any one of claims 2 to 5, wherein the plastic film comprises a polyethylene based resin.

7.  The perforated plastic film according to any one of claims 1 to 6, for use in the protection of skin.

8.  A method for producing a perforated plastic film, the method comprising:

    a step of preparing a plastic film:

        that is stretched in such a way that it is torn in a machine direction under tearing in the machine direction, is torn in a transverse direction under tearing in the transverse direction, and is torn in either the machine direction or the transverse direction under tearing in a direction at 45° to the machine direction;
        that has an acute angle formed between a tearing direction and a cut line under tearing in the direction at 45° to the machine direction of from 30 to 60°; and
        that has a tear strength exhibited under tearing in the direction at 45° to the machine direction of 10 g or less; and

    a perforation step of forming a 10 to 40000 through holes/100 cm$^2$ in the plastic film.

9.  The method for producing a perforated plastic film according to claim 8, wherein the step of preparing a plastic film comprises a step of cross-linking the plastic film with electron beam.

10. A composite product comprising a non-woven fabric and the perforated plastic film according to any one of claims 1 to 6.

11. A wound product comprising a core cylinder and the perforated plastic film according to any one of claims 1 to 6, the perforated plastic film being wound on the core cylinder.

**Patentansprüche**

1.  Perforierte Kunststofffolie, bestehend aus einer Kunststofffolie mit 10 bis 40000 durchgehenden Löchern/100 cm$^2$, wobei die perforierte Kunststofffolie eine Feuchtigkeitsdurchlässigkeit von 300 bis 3500 g/m$^2$·24 h aufweist, wobei die Kunststofffolie aus Polyethylen mit einer Gelfraktion von 10 bis 60 Massen% besteht, oder wobei die Kunststofffolie in einer Weise gereckt wird, dass sie in einer Maschinenrichtung unter Reißen in der Maschinenrichtung, in einer Querrichtung unter Reißen in der Querrichtung, und in entweder der Maschinenrichtung oder der Querrichtung unter Reißen in einer Richtung in 45° zu der Maschinenrichtung gerissen wird, wobei "gerissen in einer Maschinenrichtung" und "gerissen in einer Querrichtung" bedeutet, dass zwischen der Maschinenrichtung oder der Querrichtung und einer Schnittlinie ein spitzer Winkel von 0 bis 15° gebildet wird.

2.  Perforierte Kunststofffolie nach Anspruch 1, wobei die perforierte Kunststofffolie in einer Weise gereckt wird, dass sie in einer Maschinenrichtung unter Reißen in der Maschinenrichtung, in einer Querrichtung unter Reißen in der Querrichtung, und in entweder der Maschinenrichtung oder der Querrichtung unter Reißen in einer Richtung von 45° zu der Maschinenrichtung gerissen wird, und wobei die Kunststofffolie in einer Weise gereckt wird, so dass sie einen spitzen Winkel von 30 bis 60° aufweist, der zwischen einer Rissrichtung und einer Schnittlinie unter Reißen in der Richtung von 45° zu der Maschinenrichtung gebildet wird, und dass sie unter Reißen in die Richtung von 45° zu der Maschinenrichtung eine Reißfestigkeit von 10 g oder weniger zeigt.

3.  Perforierte Kunststofffolie nach Anspruch 1 oder Anspruch 2, wobei ein durchschnittlicher Lochdurchmesser der durchgehenden Löcher 5 bis 300 μm beträgt und ein Öffnungsbereichverhältnis in der Kunststofffolie 0,0003 bis 4,5 % beträgt.

4.  Perforierte Kunststofffolie nach einem der Ansprüche 1 bis 3, wobei ein effektives Öffnungsbereichverhältnis der durchgehenden Löcher 80 bis 100 % beträgt.

5.  Perforierte Kunststofffolie nach einem der Ansprüche 1 bis 4, wobei die Kunststofffolie einen Reibungskoeffizienten von 1,7 oder weniger aufweist.

6.  Perforierte Kunststofffolie nach einem der Ansprüche 2 bis 5, wobei die Kunststofffolie Harz auf Polyethylenbasis umfasst.

7.  Perforierte Kunststofffolie nach einem der Ansprüche 1 bis 6 zur Verwendung beim Schutz der Haut.

8.  Verfahren zum Produzieren einer perforierten Kunststofffolie, wobei das Verfahren umfasst:

    einen Schritt zum Herstellen einer Kunststofffolie,

    die in einer solchen Weise gereckt wird, dass sie in einer Maschinenrichtung unter Reißen in der Maschinenrichtung gerissen wird, in einer Querrichtung unter Reißen in der Querrichtung gerissen wird, und in entweder der Maschinenrichtung oder der Querrichtung unter Reißen in einer Richtung von 45° zu der Maschinenrichtung gerissen wird;
    die einen spitzen Winkel von 30 bis 60° aufweist, der zwischen einer Rissrichtung und einer Schnittlinie unter Reißen in der Richtung von 45° zu der Maschinenrichtung gebildet wird; und
    die eine unter Reißen in der Richtung von 45° zu der Maschinenrichtung gezeigte Reißfestigkeit von 10 g oder weniger aufweist; und

    einen Perforationsschritt, in dem in der Kunststofffolie 10 bis 40000 durchgehende Löcher/100 cm$^2$ gebildet

werden.

9. Verfahren zum Produzieren einer perforierten Kunststofffolie nach Anspruch 8, wobei der Schritt des Herstellens einer Kunststofffolie einen Schritt des Vernetzens der Kunststofffolie mit dem Elektronenstrahl umfasst.

10. Verbundprodukt, das ein Vliestextil und die perforierte Kunststofffolie nach einem der Ansprüche 1 bis 6 umfasst.

11. Wundprodukt, umfassend einen Kernzylinder und die perforierte Kunststofffolie nach einem der Ansprüche 1 bis 6, wobei die perforierte Kunststofffolie auf den Kernzylinder gewickelt ist.

**Revendications**

1. Film de plastique perforé constitué d'un film de plastique comportant de 10 à 40 000 trous traversants/100 cm$^2$, le film de plastique perforé ayant une perméabilité à l'humidité de 300 à 3 500 g/m$^2$ • 24 h,
le film de plastique se composant de polyéthylène ayant une fraction de gel de 10 à 60 %mass, ou
le film de plastique étant étiré d'une manière telle qu'il est déchiré dans un sens machine quand on le déchire dans le sens machine, dans un sens travers quand on le déchire dans le sens travers, et soit dans le sens machine, soit dans le sens travers quand on le déchire dans une direction à 45 ° par rapport au sens machine,
« déchiré dans un sens machine » et « déchiré dans un sens travers » signifiant qu'un angle aigu formé entre le sens machine ou le sens travers et une ligne de coupe est de 0 à 15 °.

2. Film de plastique perforé selon la revendication 1, le film de plastique perforé étant étiré d'une manière telle qu'il est déchiré dans un sens machine quand on le déchire dans le sens machine, dans un sens travers quand on le déchire dans le sens travers, et soit dans le sens machine, soit dans le sens travers quand on le déchire dans une direction à 45 ° par rapport au sens machine, et
le film de plastique étant étiré d'une manière telle qu'il comporte un angle aigu de 30 à 60 ° formé entre une direction de déchirure et une ligne de coupe quand on le déchire dans la direction à 45 ° par rapport au sens machine, et présente une résistance à la déchirure de 10 g ou moins quand on le déchire dans la direction à 45 ° par rapport au sens machine.

3. Film de plastique perforé selon la revendication 1 ou la revendication 2,
dans lequel un diamètre de trou moyen des trous traversants est de 5 à 300 $\mu$m, et une proportion de la surface couverte par les ouvertures dans le film de plastique est de 0,0003 à 4,5 %.

4. Film de plastique perforé selon l'une quelconque des revendications 1 à 3,
dans lequel une proportion de la surface effective couverte par les ouvertures des trous traversants est de 80 à 100 %.

5. Film de plastique perforé selon l'une quelconque des revendications 1 à 4,
le film de plastique ayant un coefficient de frottement de 1,7 ou moins.

6. Film de plastique perforé selon l'une quelconque des revendications 2 à 5,
le film de plastique comprenant une résine à base de polyéthylène.

7. Film de plastique perforé selon l'une quelconque des revendications 1 à 6, destiné à une utilisation dans la protection de la peau.

8. Procédé de fabrication d'un film de plastique perforé, le procédé comprenant :
une étape de préparation d'un film de plastique :

qui est étiré d'une manière telle qu'il est déchiré dans un sens machine quand on le déchire dans le sens machine, qu'il est déchiré dans un sens travers quand on le déchire dans le sens travers, et qu'il est déchiré soit dans le sens machine, soit dans le sens travers quand on le déchire dans une direction à 45 ° par rapport au sens machine ;
qui comporte un angle aigu de 30 à 60 ° formé entre une direction de déchirure et une ligne de coupe quand on le déchire dans la direction à 45 ° par rapport au sens machine ; et
qui présente une résistance à la déchirure de 10 g ou moins quand on le déchire dans la direction à 45 ° par rapport au sens machine ; et

une étape de perforation consistant à former 10 à 40 000 trous traversants/100 cm$^2$ dans le film de plastique.

9. Procédé de fabrication d'un film de plastique perforé selon la revendication 8,
   dans lequel l'étape de préparation d'un film de plastique comprend une étape de réticulation du film de plastique avec un faisceau d'électrons.

10. Produit composite comprenant un tissu non tissé et le film de plastique perforé selon l'une quelconque des revendications 1 à 6.

11. Produit enroulé comprenant un cylindre de base et le film de plastique perforé selon l'une quelconque des revendications 1 à 6, le film de plastique perforé étant enroulé sur le cylindre de base.

## Figure 1

Tear test at a 45° angle

45°

TD ←

+θ

↓ MD

$\theta = +45°$ ···Cut in TD direction
$\theta = -45°$ ···Cut in MD direction
$\theta = \ 0°$ ···Cut at a 45° angle

· JIS K 7128 Elmendorf tear strength tester
· Sample size 60mm×60mm
· Slit length 10mm

## Figure 2

Figure 3

Figure 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 62275456 A
- JP 5261145 A
- JP 63183055 A
- US 4499896 A
- JP S62148246 A
- JP 2014237810 A
- JP 2007089493 A
- JP H0586216 A
- JP 1995281 A
- JP 2014229488 A
- JP 2014229490 A